# EUROPEAN PATENT SPECIFICATION

(11) **EP 2 544 895 B1**
(45) Date of publication and mention of the grant of the patent: **01.10.2014**
(21) Application number: 11707949.1
(22) Date of filing: 11.02.2011
(51) Int. Cl.: D04H 3/03, B32B 5/26, D01F 6/30, B32B 27/32, B29C 49/00, B32B 37/15, B32B 3/28, C08L 23/04, C08L 23/08, C08L 23/10, C08L 23/14, B32B 27/08, D01D 5/098, D04H 3/16

(54) **ELASTIC MELTBLOWN LAMINATE CONSTRUCTIONS AND METHODS FOR MAKING SAME**
ELASTISCHE SCHMELZGEBLASENE LAMINATKONSTRUKTIONEN UND HERSTELLUNGSVERFAHREN DAFÜR
CONSTRUCTIONS DE STRATIFIÉS ÉLASTIQUES EXTRUDÉS-SOUFFLÉS ET LEURS PROCÉDÉS DE FABRICATION

(30) Priority: 12.03.2010 US 723317; 12.03.2010 US 723336
(43) Date of publication of application: 16.01.2013
(73) Proprietor: ExxonMobil Chemical Patents Inc., Baytown, TX 77520 (US)
(72) Inventor: WESTWOOD, Alistair, Duncan, Kingwood TX 77345 (US); RICHESON, Galen, Charles, Humble TX 77346 (US)
(74) Representative: Mareschal, Anne
(86) International application number: PCT/US2011/024589
(87) International publication number: WO 2011/112311

(56) References cited:
- EP-A1- 1 066 961
- WO-A1-01/00917
- WO-A1-2011/041575
- US-A- 4 209 563
- US-A- 5 723 217
- US-A1- 2008 182 468

## Description

### FIELD OF THE INVENTION

This disclosure relates to elastic meltblown fibers containing propylene-α-olefin copolymers, as well as elastic meltblown fabrics made therefrom, multilayer constructions made therefrom, and methods for making same.

### BACKGROUND OF THE INVENTION

The market desires a highly elastic, breathable, nonwoven fabric with the necessary aesthetic qualities that requires no form of mechanical activation. Existing products are complex laminates made of an elastic film, typically a styrenic block copolymer ("SBC") or polyurethane that has polyolefin skins coextruded onto the film to prevent blocking, and nonwovens in order to provide the correct aesthetic (a soft, fluffy, cushion-like texture) and in certain constructions a hot melt glue layer to bond the nonwoven to either side of the elastic film. These types of constructions, once formed, are often not elastic due to the constraining influence of the inelastic components such as the polyolefin skin layers and nonwoven facing layers.

In order to remove the constraining influence of non-elastic elements, many composites require a mechanical stretching or activation process in order to stretch or break the non-elastic components. The mechanical stretching removes the constraints and creates an elastic composite controlled by the SBC film. Furthermore, such composites require the film to be apertured in order to make these laminates breathable. This process involves the controlled puncturing/tearing of the film with the associated concerns for film failure and increased scrap rates.

Recently, film composites have arrived on the market that do not require mechanical activation. These products still use a SBC film layer with a highly extensible spunlaced layer attached to either side of the film using thin lines of hot melt glue. The regions between the glued areas are not constrained, and are therefore elastic, because the film does not have a coextruded skin and the nonwoven is extensible and non-restraining. However, these products are not breathable, require adhesives, and like all of the film laminate products are costly to produce.

A solution to the problem is to make highly extensible (> 500% Ultimate Elongation with a low tensile force) nonwoven fabrics with meltblowing systems capable of melt blowing low melt flow rate elastomers to produce *in situ* a laminate ("construction") of extensible nonwovens and a highly elastic, breathable, high molecular weight meltblown fabric without the need for adhesive or external thermal or mechanical bonding processes.

Some relevant disclosures may include European Patent No. 1 712 351 A; and U.S. Patent No(s): 4,380,570; 5,476,616; 5,804,286; 5,921,973; 6,342,565; 6,417,121; 6,444,774; 6,506,698; and U.S. Publication No(s): 2003/0125696; 2005/0130544 A1; 2006/0172647; and R. Zhao, "Melt Blowing Polyoxymethylene Copolymer" in INT'L NONWOVENS J., pp. 19-24 (Summer 2005), and EP1066961.

### SUMMARY OF THE INVENTION

Multilayer meltblown composites, articles made therefrom, and methods for making the same are provided. In at least one specific embodiment, the meltblown composite can include a first meltblown layer comprising one or more resins having an Ultimate Elongation (UE) of from 50% to 250%, as measured according to ASTM D412; and a second meltblown layer comprising a propylene-α-olefin copolymer having an ethylene content of 5 wt% to 20 wt%; a MFR (ASTM-1238D, 2.16 kg, 230°C) of 10 g/10 min to 30 g/10 min; and a heat of fusion of 75 J/g or less.

In at least one specific embodiment, the method includes meltblowing a first material to form a first meltblown layer; and meltblowing a second material on at least a portion of the first melt blown layer, wherein the second meltblown layer comprises a propylene-α-olefin copolymer having an ethylene content of 5 wt% to 20 wt%; a MFR (ASTM-1238D, 2.16 kg, 230°C) of 10 g/10 min to 30 g/10 min; and a heat of fusion of 75 J/g or less.

In at least one specific embodiment, an article incorporating as one or more components, a multilayer meltblown composite is provided. The multilayer meltblown composite can include a first meltblown layer comprising one or more resins having an Ultimate Elongation (UE) of from 50% to 250%, as measured according to ASTM D412; and a second meltblown layer comprising one or more resins having an Ultimate Elongation (UE) of 200% or more, as measured according to ASTM D412, wherein at least one resin comprises a propylene-α-olefin copolymer having an ethylene content of 5 wt% to 20 wt%; a MFR (ASTM-1238D, 2.16 kg, 230°C) of 10 g/10 min to 30 g/10 min; and a heat of fusion of 75 J/g or less.

### BRIEF DESCRIPTION OF THE DRAWINGS

Figure 1 depicts a schematic view of an illustrative meltblowing system for making a multilayer meltblown composite, according to one or more embodiments described.
Figure 2 depicts an enlarged schematic view of an illustrative die assembly, according to one or more embodiments described.
Figure 3 depicts a schematic of an illustrative meltblowing system for making a multilayer meltblown laminate or composite, according to one or more embodiments described. As depicted, the dies and collection surfaces can be vertically disposed.
Figure 4 depicts a schematic of another illustrative meltblowing system for making a multilayer meltblown laminate or composite, according to one or more embodiments described. As depicted, the dies and collection surfaces can be horizontally disposed.
Figure 5 graphically depicts the two sample sets listed in Tables 1 and 2 with the propylene-α-olefin copolymer ("PCP") Basis Weight per Total Fabric Basis Weight on the x-axis and the peak force @ 100% / Retractive Force @ 50% on the y-axis.

### DETAILED DESCRIPTION

It has been discovered that the expression, y=Ax^{b}, where A is 3, b is zero to infinity, x is the peak load @ 100%/retractive force @ 50% and y is the facing layer constraint, is found to describe the general elastic performance of multilayer constructions or laminates containing one or more propylene-α-olefin copolymers made using the multilayer forming process provided herein. Surprisingly, other resins and other forming processes exhibit different values for this expression.

Not wishing to be bound by theory, it is shown that different facing layers introduce different levels of constraint as defined by the value of y. As the ratio of the propylene-α-olefin copolymer content to the overall fabric basis weight decreases as defined by the x value (i.e., less propylene-α-olefin copolymer for a constant facing layer construct) the ratio of peak force to retractive force increases. As the propylene-α-olefin copolymer content decreases for a constant facing layer construct the level of constraint follows the general power law relationship described. In other words, a change in the nature of the facing layer changes the level of constraint; however, the general power law holds just the power changes.

It has also been discovered that the less constraint that the facing layer imparts, the lower the "b" value. Conversely, the greater the constraint imposed by the facing layer, the greater the "b" value. The two extreme cases being, if the facing layer imposed no constraint whatsoever on the laminate then b = 0 and the y value would equal 3 just like the value of the neat propylene-α-olefin copolymer with no facing layer. The other extreme would be if the facing layers were highly constraining stiff plastic layers that once stretched to 100% did not allow any recovery of more than 50%. Therefore, if the retractive force at 50% is 0, the ratio is infinite and b approaches infinity.

The elastic meltblown fibers, fabrics and multilayer constructions or laminates made therefrom can include at least one layer of elastic meltblown fabric. The elastic meltblown fabrics and multilayer constructions or laminates made therefrom can also include at least one layer of extensible meltblown fabric. Preferably, the meltblown layers are disposed adjacent one another. It is envisaged, however, that one or more spunlace, spunbond, spunlaid, textiles, air laid, pulp, woven, super-absorbent polymer(s) ("SAP"), or films can be disposed on or between the meltblown layers.

Each meltblown layer can include one or more resins that are the same or different. Each resin can be an extensible resin, an elastic resin, or an inelastic resin. Suitable resins for any given layer can also be a blend of two or more resins, where each resin in extensible, inelastic, or elastic, such that the resulting blend can be extensible, inelastic, or elastic depending on the chosen resins, and their relatives amounts.

As used herein, a "composite" or "fabric" is a structure, preferably flat but bendable and otherwise formable, having a thickness such that it impedes, but does not stop, the passage of air, the structure made from fibers that are bound together through chemical bonding, melt adhesion or weaving (mechanical linkage) such that they form the structure. As used herein, a "fiber" is a material whose length is very much greater than its diameter or breadth: the average diameter is on the order of 5 to 250 µm, and includes natural and/or synthetic substances.

As used herein, materials, resins, fibers, and/or fabrics referred to as being "elastic" are those that can recover at least 70% after 100% deformation. As used herein, materials, resins, fibers, and/or fabrics referred to as being "inelastic" are those that can recover less than 20% after 100% deformation. As used herein, materials, resins, fibers, and/or fabrics referred to as being "extensible" are those that can recover 20% to 70% after 100% deformation, as determined by ASTM D412. Extensible materials and fabrics are well known in the art and are those formed, in one instance, from a material that is extensible or by mechanically distorting or twisting a fabric (natural or synthetic) such as described in US Patent No. 5,523,141.

Suitable resins can be or include cellulosics, nylons, polyacetals, polyalkylene naphthalates, polyesters, co-polyesters, polyurethane, polyamids, polyamides, polyolefins, polyolefin homopolymers, polyolefin copolymers, acrylic, and blends thereof. Except as stated otherwise, the term "copolymer" means a polymer derived from two or more monomers (including terpolymers, tetrapolymers, etc., that can be arranged in a random, block, or grafted distribution), and the term "polymer" refers to any carbon-containing compound having repeat units from one or more different monomers.

Preferred cellulosic materials include rayon and viscose. A preferred polyacetal is polyoxymethylene copolymer. Preferred polyesters include polyolefin-terephthalates and polyalkylene terephthalates, such as poly(ethylene terephthalate) (PET), poly(butylene terephthalate) (PBT), and poly(cyclohexane dimethylene terephthalate) (PCT).

Preferred polyolefins can be prepared from mono-olefin monomers including, but not limited to, monomers having 2 to 8 carbon atoms, such as ethylene, propylene, 1-butene, isobutylene, 1-pentene, 1-hexene, 1-octene, 3-methyl-1-pentene, 4-methyl-1-pentene, 5-methyl-1-hexene, mixtures thereof and copolymers thereof with (meth)acrylates and/or vinyl acetates. Other suitable polyolefins can include one or more propylene homopolymers (100 wt% propylene-derived units), propylene copolymers, propylene-α-olefin copolymers, polypropylene impact copolymers (ICP), random copolymers (RCP) linear low density polyethylene, high density polyethylene, low density polyethylene, ethylene block copolymers (e.g., Infuse™ olefin block copolymers), styrenic block copolymers (e.g., Kraton™ styrenic copolymers), ethylene vinylacetates, urethanes, polyesters, and blends thereof. Certain specific extensible resins can include polyacrylonitrile, polybutylene terephthalate, polyethylene terephthalate (PET), polycyclohexylenedimethylene terephthalate (PCT), polyamide and/or acrylic.

As used herein, "polypropylene" refers to a propylene homopolymer, or a copolymer of propylene, or some mixture of propylene homopolymers and copolymers. In certain embodiments, the polypropylene described herein is predominately crystalline, thus the polypropylene may have a melting point (Tₘ) greater than 110°C or 115°C or 130°C. The term "crystalline," as used herein, characterizes those polymers which possess high degrees of inter-and intra-molecular order. In certain embodiments the polypropylene has a heat of fusion (H_{f}) greater than 60 J/g or 70 J/g or 80 J/g, as determined by DSC analysis. The heat of fusion is dependent on the composition of the polypropylene; the thermal energy for the highest order of polypropylene is estimated at 189 J/g that is, 100% crystallinity is equal to a heat of fusion of 189 J/g. A polypropylene homopolymer will have a higher heat of fusion than a copolymer or blend of homopolymer and copolymer.

In certain embodiments, the polypropylene(s) can be isotactic. Isotacticity of the propylene sequences in the polypropylenes can be achieved by polymerization with the choice of a desirable catalyst composition. The isotacticity of the polypropylenes as measured by ¹³C NMR, and expressed as a meso diad content is greater than 90% (meso diads [m] > 0.90) or 95% or 97% or 98% in certain embodiments, determined as in US 4,950,720 by ¹³C NMR. Expressed another way, the isotacticity of the polypropylenes as measured by ¹³C NMR, and expressed as a pentad content, is greater than 93% or 95% or 97% in certain embodiments.

The polypropylene can vary widely in composition. For example, substantially isotactic polypropylene homopolymer or propylene copolymer containing equal to or less than 10 wt% of other monomer, that is, at least 90 wt% propylene can be used. Further, the polypropylene can be present in the form of a graft or block copolymer, in which the blocks of polypropylene have substantially the same stereoregularity as the propylene-α-olefin copolymer (described below) so long as the graft or block copolymer has a sharp melting point above 110°C or 115°C or 130°C, characteristic of the stereoregular propylene sequences.

The polypropylene can be a combination of homopolypropylene, and/or random, and/or block copolymers as described herein. When the polypropylene is a random copolymer, the percentage of the α-olefin derived units in the copolymer is, in general, up to 5 wt% of the polypropylene, 0.5 wt% to 5 wt% in another embodiment, and 1 wt% to 4 wt% in yet another embodiment. The preferred comonomer derived from ethylene or α-olefins containing 4 to 12 carbon atoms. One, two or more comonomers can be copolymerized with propylene. Exemplary α-olefins may be selected from the group consisting of ethylene; 1-butene; 1-pentene-2-methyl-1-pentene-3-methyl-1-butene; 1-hexene-3-methyl-1-pentene-4-methyl-1-pentene-3,3-dimethyl-1-butene; 1-heptene; 1-hexene; 1-methyl-1-hexene; dimethyl-1-pentene; trimethyl-1-butene; ethyl-1-pentene; 1-octene; methyl-1-pentene; dimethyl-1-hexene; trimethyl-1-pentene; ethyl-1-hexene; 1-methylethyl-1-pentene; 1-diethyl-1-butene; propyl-1-pentene; 1-decene; methyl-1-nonene; 1-nonene; dimethyl-1-octene; trimethyl-1-heptene; ethyl-1-octene; methylethyl-1-butene; diethyl-1-hexene; 1-dodecene and 1-hexadodecene.

The weight average molecular weight (Mw) of the polypropylene can be between 50,000 g/mol to 3,000,000 g/mol, or from 90,000 g/mol to 500,000 g/mol in another embodiment, with a molecular weight distribution (MWD, Mw/Mn) within the range from 1.5 to 2.5; or 3.0 to 4.0; or 5.0 to 20.0. The polypropylene can have an MFR (2.16kg/ 230°C) ranging of from 10 dg/min to 15 dg/min; or 18 dg/min to 30 dg/min; or 35 dg/min to 45 dg/min; or 40 dg/min to 50 dg/min.

The term "random polypropylene" ("RCP") as used herein broadly means a single phase copolymer of propylene having up to 9 wt%, preferably 2 wt% to 8 wt% of an alpha olefin comonomer. Preferred alpha olefin comonomers have 2 carbon atoms, or from 4 to 12 carbon atoms. Preferably, the alpha olefin comonomer is ethylene.

The propylene impact copolymers ("ICP") is heterogeneous and can include a first phase of 70 to 95 wt% homopolypropylene and a second phase of from 5 to 30 wt% ethylene-propylene rubber, based on the total weight of the impact copolymer. The propylene impact copolymer can include 78 to 95 wt% homopolypropylene and from 5 to 22 wt% ethylene-propylene rubber, based on the total weight of the impact copolymer. In certain embodiments, the propylene-based polymer can include from 90 wt% to 95 wt% homopolypropylene and from 5 wt% to 10 wt% ethylene-propylene rubber, based on the total weight of the impact copolymer.

There is no particular limitation on the method for preparing the polypropylenes described herein. However, for example, the polymer is a propylene homopolymer obtained by homopolymerization of propylene in a single stage or multiple stage reactor. Copolymers may be obtained by copolymerizing propylene and ethylene or an α-olefin having from 4 to 20 carbon atoms in a single stage or multiple stage reactor. Polymerization methods include, but are not limited to, high pressure, slurry, gas, bulk, or solution phase, or a combination thereof, using any suitable catalyst such as traditional Ziegler-Natta catalyst or a single-site, metallocene catalyst system, or combinations thereof including bimetallic (i.e., Ziegler-Natta and metallocene) supported catalyst systems.

Exemplary commercial polypropylenes include the family of Achieve™ polymers (ExxonMobil Chemical Company, Baytown, TX). The Achieve polymers are produced using metallocene catalyst systems. In certain embodiments, the metallocene catalyst system produces a narrow molecular weight distribution polymer. The MWD is typically in the range of 1.5 to 2.5. However, a broader MWD polymer may be produced in a process with multiple reactors. Different MW polymers can be produced in each reactor to broaden the MWD. Achieve polymer such as Achieve 3854, a homopolymer having an MFR of 24 dg/min can be used as a blend component described herein. Alternatively, an Achieve polymer such as Achieve 6936G1, a 1,550 dg/min MFR homopolymer, can be used as a blend component described herein. Other polypropylene random copolymer and impact copolymer may also be used. The choice of polypropylene MFR can be used as means of adjusting the final MFR of the blend, especially the facing layer composition. Any of the polypropylenes described herein can be modified by controlled rheology to improve spinning performance as is known in the art.

The "propylene-α-olefin copolymer" or "PCP" is a copolymer of propylene-derived units and one or more units derived from ethylene or a C₄-C₁₀ α-olefin and optionally one or more diene-derived units, and are relatively elastic and/or form nonwoven fibers and fabrics that are elastic (Ultimate Elongation from greater than 500%). The overall comonomer content of the copolymer is within the range from 5 to 35 wt% in one embodiment. In some embodiments, where more than one comonomer is present, the amount of a particular comonomer may be less than 5 wt%, but the combined comonomer content is from greater than 5 wt%. The propylene-α-olefin copolymers may be described by any number of different parameters, and those parameters may include a numerical range made up of any desirable upper limit with any desirable lower limit as described herein for the propylene-α-olefin copolymers.

The propylene-α-olefin copolymer may be a terpolymer of propylene, block copolymer (the comonomer-derived units occur along long sequences), impact copolymer of propylene, random polypropylene, random copolymer (the comonomer-derived units are randomly distributed along the polymer backbone), or mixtures thereof. The presence of randomness or "blocky-ness" in a copolymer can be determined by ¹³C NMR as is known in the art and described in, for example, 18 J. Poly. Sci.: Poly. Lett. Ed., pp. 389-394 (1980).

In certain embodiments, the propylene-α-olefin copolymer can include ethylene or C₄-C₁₀ α-olefin-derived units (or "comonomer-derived units") within the range from 5 wt%; or 7 wt%; or 8 wt%; or 10 wt% to 18 wt%; or 20 wt%; or 25 wt%; or 32 wt%; or 35 wt% of the copolymer. The propylene-α-olefin copolymer may also include two different comonomer-derived units. Also, these copolymers and terpolymers may include diene-derived units as described below. In a particular embodiment, the propylene-α-olefin copolymer includes propylene-derived units and comonomer units selected from ethylene, 1-butene, 1-hexene, and 1-octene. And in a more particular embodiment, the comonomer is ethylene, and thus the propylene-α-olefin copolymer is a propylene-ethylene copolymer.

In one embodiment, the propylene-α-olefin copolymer includes from less than 10 wt% or 8 wt% or 5 wt% or 3 wt% of the copolymer, of diene derived units (or "diene"), and within the range from 0.1 wt%; or 0.5 wt%; or 1 wt% to 5 wt%; or 8 wt%; or 10 wt%, in yet another embodiment. Suitable dienes include for example: 1,4-hexadiene; 1,6-octadiene; 5-methyl-1,4-hexadiene; 3,7-dimethyl-1,6-octadiene; dicyclopentadiene (DCPD); ethylidiene norbornene (ENB); norbomadiene; 5-vinyl-2-norbornene (VNB); and combinations thereof. The diene, if present, is most preferably ENB.

In certain embodiments, the propylene-α-olefin copolymers have a triad tacticity of three propylene units, as measured by ¹³C NMR, from greater than 75%; or 80%; or 82%; or 85%; or 90%. In one embodiment, the triad tacticity is within the range from 50 to 99 %; and from 60 to 99%, in another embodiment; and from 75 to 99%, in yet another embodiment; and from 80 to 99%, in yet another embodiment; and from 60 to 97%, in yet another embodiment. Triad tacticity is determined as follows: The tacticity index, expressed herein as "m/r", is determined by ¹³C NMR. The tacticity index m/r is calculated as defined by H. N. Cheng, Vol. 17, MACROMOLECULES, pp. 1950-1955 (1984). The designation "m" or "r" describes the stereochemistry of pairs of contiguous propylene groups, "m" referring to meso and "r" to racemic. An m/r ratio of 1.0 generally describes a syndiotactic polymer, and an m/r ratio of 2.0 an atactic material. An isotactic material theoretically may have a ratio approaching infinity, and many by-product atactic polymers have sufficient isotactic content to result in ratios from greater than 50. Embodiments of the propylene-α-olefin copolymer have a tacticity index m/r within the range from 4 or 6 to 8 or 10 or 12.

In certain embodiments, the propylene-α-olefin copolymers have a heat of fusion (H_{f}), determined according to the Differential Scanning Calorimetry (DSC) procedure described herein, within the range from 0.5; or 1; or 5 J/g, to 35; or 40; or 50; or 65; or 75 J/g. In certain embodiments, the H_{f} value is less than 75; or 65; or 55 J/g.

In certain embodiments, the propylene-α-olefin copolymers have a percent crystallinity within the range from 0.5 to 40%, and from 1 to 30% in another embodiment, and from 5 to 25% in yet another embodiment, wherein "percent crystallinity" is determined according to the DSC procedure described herein. (The thermal energy for the highest order of polypropylene is estimated at 189 J/g (i.e., 100% crystallinity is equal to 189 J/g)). In another embodiment, the propylene-α-olefin copolymer has a percent crystallinity from less than 40% or 25% or 22% or 20%.

In certain embodiments, the propylene-α-olefin copolymers have a single peak melting transition as determined by DSC; in certain embodiments the propylene-α-olefin copolymer has a primary peak melting transition from less than 90°C, with a broad end-of melt transition from greater than about 110°C. The peak "melting point" (Tₘ) is defined as the temperature of the greatest heat absorption within the range of melting of the sample. However, the propylene-α-olefin copolymer may show secondary melting peaks adjacent to the principal peak, and/or the end-of-melt transition, but for purposes herein, such secondary melting peaks are considered together as a single melting point, with the highest of these peaks being considered the Tₘ of the propylene-α-olefin copolymer. The propylene-α-olefin copolymers have a peak melting temperature (Tₘ) from less than 70; or 80; or 90; or 100; or 105°C in certain embodiments; and within the range from 10; or 15; or 20; or 25 to 65; or 75; or 80; or 95; or 105°C in other embodiments.

The procedure for DSC determinations is as follows. 0.5 grams of polymer is weighed out and pressed to a thickness of 15-20 mils (381-508 microns) at 140°C -150°C, using a "DSC mold" and Mylar™ as a backing sheet. The pressed pad is allowed to cool to ambient temperature by hanging in air (the Mylar was not removed). The pressed pad is annealed at room temperature (about 23°C-25°C) for about 8 days. At the end of this period, a 15-20 mg disc is removed from the pressed pad using a punch die and placed in a 10 microliter aluminum sample pan. The sample is placed in a differential scanning calorimeter (Perkin Elmer Pyris 1 Thermal Analysis System) and cooled to - 100°C. The sample is heated at 10°C/min to attain a final temperature of 165°C. The thermal output, recorded as the area under the melting peak of the sample, is a measure of the heat of fusion and can be expressed in Joules per gram (J/g) of polymer and automatically calculated by the Perkin Elmer System. Under these conditions, the melting profile shows two (2) maxima, the maxima at the highest temperature was taken as the melting point within the range of melting of the sample relative to a baseline measurement for the increasing heat capacity of the polymer as a function of temperature.

In certain embodiments, the propylene-α-olefin copolymers have a density within the range from 0.840 g/cm³ to 0.920 g/cm³; and from 0.845 g/cm³ to 0.900 g/cm³, in another embodiment; and from 0.850 g/cm³ to 0.890 g/cm³, in yet another embodiment, the values measured at room temperature per the ASTM D-1505 test method.

In certain embodiments, the propylene-α-olefin copolymers have a Shore A hardness (ASTM D2240) within the range from 10; or 20 to 80; or 90 Shore A. In yet another embodiment, the propylene-α-olefin copolymers possess an Ultimate Elongation (ASTM-D412) greater than 500%, 1,000% or 2,000%. The propylene-α-olefin copolymers can also have an Ultimate Elongation (ASTM-D412) ranging from a low of about 300%; 400%; or 500% to a high of about 800%; 1,200%; 1,800%; 2,000%; or 3,000%.

In certain embodiments, the propylene-α-olefin copolymers have a weight average molecular weight (Mw) value within the range from 20,000 to 5,000,000 g/mole; and from 50,000 to 1,000,000 g/mole, in another embodiment; and from 70,000 to 400,000 g/mole, in yet another embodiment. In another embodiment, the propylene-α-olefin copolymers have a number average molecular weight (Mn) value within the range from 4,500 to 2,500,000 g/mole; and from 20,000 to 250,000 g/mole, in yet another embodiment; and from 50,000 to 200,000 g/mole, in yet another embodiment. In yet another embodiment, the propylene-α-olefin copolymers have a z-average molecular weight (Mz) value within the range from 20,000 to 7,000,000 g/mole; and from 100,000 to 700,000 g/mole, in another embodiment; and from 140,000 to 500,000 g/mole, in yet another embodiment.

In certain embodiments, a desirable molecular weight (and hence, a desirable MFR) is achieved by visbreaking the propylene-α-olefin copolymers. The "visbroken propylene-α-olefin copolymers" (also known in the art as "controlled rheology" or "CR") is a copolymer that has been treated with a visbreaking agent such that the agent breaks apart the polymer chains. Non-limiting examples of visbreaking agents include peroxides, hydroxylamine esters, and other oxidizing and free-radical generating agents. Stated another way, the visbroken copolymer may be the reaction product of a visbreaking agent and the copolymer. In particular, a visbroken propylene-α-olefin copolymer is one that has been treated with a visbreaking agent such that its MFR is increased, in one embodiment by at least 10%, and at least 20% in another embodiment relative to the MFR value prior to treatment.

In certain embodiments, the molecular weight distribution (MWD) of the propylene-α-olefin copolymers is within the range from 1.5; or 1.8; or 2.0 to 3.0; or 3.5; or 4.0; or 5.0; or 10.0, in particular embodiments. Techniques for determining the molecular weight (Mn, Mz and Mw) and molecular weight distribution (MWD) are as follows, and as by Verstate et al., Vol. 21, MACROMOLECULES, pp. 3360-3371 (1988). Conditions described herein govern over published test conditions. Molecular weight and molecular weight distribution are measured using a Waters 150 gel permeation chromatograph equipped with a Chromatix KMX-6 on-line light scattering photometer. The system was used at 135°C with 1,2,4-trichlorobenzene as the mobile phase. Showdex™ (Showa-Denko America, Inc.) polystyrene gel columns 802, 803, 804 and 805 are used. This technique is discussed in LIQUID CHROMATOGRAPHY OF POLYMERS AND RELATED MATERIALS III, pp. 207-234 (J. Cazes ed., Marcel Dekker, 1981). No corrections for column spreading were employed; however, data on generally accepted standards, for example, National Bureau of Standards, Polyethylene (SRM 1484) and anionically produced hydrogenated polyisoprenes (an alternating ethylene-propylene copolymer) demonstrate that such corrections on Mw/Mn or Mz/Mw are less than 0.05 units. Mw/Mn was calculated from an elution time-molecular weight relationship whereas Mz/Mw was evaluated using the light scattering photometer. The numerical analyses can be performed using the commercially available computer software GPC2, MOLWT2 available from LDC/Milton Roy-Riviera Beach, Fla.

The propylene-α-olefin copolymers described herein can be produced using any catalyst and/or process known for producing polypropylenes. In certain embodiments, the propylene-α-olefin copolymers can include copolymers prepared according to the procedures in International Publication No. WO 02/36651, U.S. Patent No. 6992158, and/or International Publication No. WO 00/01745. Preferred methods for producing the propylene-α-olefin copolymers are found in U.S. Patent Publication No. 2004/0236042 and U.S. Patent No. 6,881,800. Preferred propylene-α-olefin copolymers are available commercially under the trade names VISTAMAXX™ (ExxonMobil Chemical Company, Houston, TX, USA) and VERSIFY™ (The Dow Chemical Company, Midland, Michigan, USA), certain grades of TAFMER™ XM or NOTIO™ (Mitsui Company, Japan), certain grades of LMPO™ from Idemitsu, or certain grades of SOFTELL™ (Lyondell Basell Polyolefine GmbH, Germany).

In one or more embodiments, the meltblown resin can be or include: natural rubber (NR); synthetic polyisoprene (IR); butyl rubber (copolymer of isobutylene and isoprene, IIR); halogenated butyl rubbers (chloro-butyl rubber (CIIR); bromo-butyl rubber (BIIR)); polybutadiene (BR); styrene-butadiene rubber (SBR); SEBS block copolymers; SIS block copolymers; SBS block copolymers; ethylene-octene block copolymers; ethylene-octene copolymers; ethylene-hexene copolymers; ethylene-butene copolymers; nitrile rubber; hydrogenated nitrile rubbers; chloroprene rubber (CR); polychloroprene; neoprene; EPM (ethylene-propylene rubber); EPDM rubbers (ethylene-propylene-diene rubber); epichlorohydrin rubber (ECO); polyacrylic rubber (ACM, ABR); silicone rubber; fluorosilicone rubber; fluoroelastomers; perfluoroelastomers; polyether block amides (PEBA); chlorosulfonated polyethylene (CSM); ethylene-vinyl acetate (EVA); thermoplastic elastomers (TPE); thermoplastic vulcanizates (TPV); thermoplastic polyurethane (TPU); thermoplastic olefins (TPO); polysulfide rubber; or blends of any two or more of these elastomers. In at least one specific embodiment, the elastic resin is or includes one or more polyolefin polymers. The term "polyolefin polymers" refers to homopolymers or copolymers of α-olefins having less than 40% crystallinity, or a heat of fusion (H_{f}) of less than 75 J/g.

In certain embodiments, the meltblown resin can be or include one or more metallocene polyethylenes ("mPE's"), including one or more mPE homopolymers or copolymers. The mPE homopolymers or copolymers may be produced using mono- or bis-cyclopentadienyl transition metal catalysts in combination with an activator of alumoxane and/or a non-coordinating anion in solution, slurry, high pressure or gas phase. The catalyst and activator may be supported or unsupported and the cyclopentadienyl rings may be substituted or unsubstituted. Several commercial products produced with such catalyst/activator combinations are commercially available from ExxonMobil Chemical Company in Baytown, Texas under the tradename EXACT™. For more information on the methods and catalysts/activators to produce such mPE homopolymers and copolymers see PCT Patent Publication No(s): WO 94/26816; WO 92/00333; WO 91/09882; WO 94/03506; and WO 94/03506; European Patent No(s). 0 277 003; 0 129 368; 0 520 732; 0 426 637; 0 573 403; 0 520 732; 0 495 375; 0 500 944; 0 570 982; and 0 277004; U.S. Patent No(s). 5,153,157; 5,198,401; 5,240,894; 5,324,800; 5,264,405; 5,096,867; 5,507,475; 5,055,438; and 5,017,714; and Canadian Patent No. 1,268,753.

In certain embodiments, the meltblown resin can be or include one or more termonomers and tetramonomers which may be one or more C₃ to C₂₀ olefins, any C₄ to C₂₀ linear, cyclic or branched dienes or trienes and any styrenic monomers such as styrene, alpha-methyl styrene, or para-methyl styrene. Preferred examples include butadiene, pentadiene, cyclopentadiene, hexadiene, cyclohexadiene, heptadiene, octadiene, nonadiene, norbornene, vinyl norbornene, ethylidene norbornene, isoprene and heptadiene.

The C₃-C₂₀ and C₄-C₂₀ olefins can be any polymerizable olefin monomer and are preferably a linear, branched or cyclic olefin, even more preferably an alpha-olefin. Examples of suitable olefins include: propylene; butene; isobutylene; pentene; isopentene; cyclopentene; hexene; isohexene; cyclohexene; heptene; isoheptene; cycloheptene; octene; isooctene; cyclooctene; nonene; cyclononene; decene; isodecene; dodecene; isodecene; 4-methyl-pentene-1; 3-methyl-pentene-1; and 3,5,5-trimethyl hexene-1. Suitable comonomers also include dienes, trienes, and styrenic monomers. Preferred examples include: styrene; alpha-methyl styrene; para-alkyl styrene (such as para-methyl styrene); hexadiene; norbornene; vinyl norbornene; ethylidene norbornene; butadiene; isoprene; heptadiene; octadiene; and cyclopentadiene. Preferred comonomers for the copolymer of ethylene are propylene, butene, hexene and/or octene.

In certain embodiments, the meltblown resin can include one or more polyalphaolefins (PAOs). PAOs are high purity hydrocarbons, with a fully paraffinic structure and a high degree of branching. Suitable PAOs are liquids with a pour point of - 10°C or less and a kinematic viscosity at 100°C (KV100°C) of 3 cSt or more. Such PAOs can include C₁₅-C₁₅₀₀ (preferably C₂₀-C₁₀₀₀, preferably C₃₀-C₈₀₀, preferably C₃₅-C₄₀₀, most preferably C₄₀-C₂₅₀) oligomers (such as dimers, trimers, etc.) of C₃-C₂₄ (preferably C₅-C₁₈, preferably C₆-C₁₄, preferably C₈-C₁₂) alpha-olefins, preferably linear alpha-olefins (LAOs), provided that C₃ and C₄ alpha-olefins are present at 30 wt% or less (preferably 20 wt% or less, preferably 10 wt% or less, preferably 5 wt% or less). Suitable LAOs include: propylene; 1-butene; 1-pentene; 1-hexene; 1-heptene; 1-octene; 1-nonene; 1-decene; 1-undecene; 1-dodecene; 1-tridecene; 1-tetradecene; 1-pentadecene; 1-hexadecene; and blends thereof.

In one or more embodiments, a single LAO is used to prepare the oligomers. A preferred embodiment involves the oligomerization of 1-octene or 1-decene, preferably 1-decene. In one or more embodiments, the PAO is or includes oligomers of two or more C₃-C₁₈ LAOs, to make 'bipolymer' or 'terpolymer' or higher-order copolymer combinations, provided that C₃ and C₄ LAOs are present 30 wt% or less (preferably 20 wt% or less, preferably 10 wt% or less, preferably 5 wt% or less). A preferred embodiment involves oligomerization of a mixture of LAOs selected from C₆-C₁₈ LAOs with even carbon numbers. Another preferred embodiment involves oligomerization of 1-octene, 1-decene, and 1-dodecene.

In one or more embodiments, the PAO comprises oligomers of a single alpha-olefin species having a carbon number of 5 to 24 (preferably 6 to 18, more preferably 8 to 12, most preferably 10). In one or more embodiments, the PAO comprises oligomers of mixed alpha-olefins (i.e., two or more alpha-olefin species), each alpha-olefin having a carbon number of 5 to 24 (preferably 6 to 18, preferably 8 to 12). In one or more embodiments, the PAO comprises oligomers of mixed alpha-olefins (i.e., involving two or more alpha-olefin species) where the weighted average carbon number for the alpha-olefin mixture is 6 to 14 (preferably 8 to 12, preferably 9 to 11).

In one or more embodiments, the PAO or blend of PAOs has a number-average molecular weight (Mₙ) of from 400 to 15,000 g/mol (preferably 400 to 12,000 g/mol, preferably 500 to 10,000 g/mol, preferably 600 to 8,000 g/mol, preferably 800 to 6,000 g/mol, preferably 1,000 to 5,000 g/mol). In one or more embodiments, the PAO or blend of PAOs has a Mₙ greater than 1,000 g/mol (preferably greater than 1,500 g/mol, preferably greater than 2,000 g/mol, preferably greater than 2,500 g/mol).

In one or more embodiments, the PAO or blend of PAOs has a KV100°C of 3 cSt or more (preferably 4 cSt or more, preferably 5 cSt or more, preferably 6 cSt or more, preferably 8 cSt or more, preferably 10 cSt or more, preferably 20 cSt or more, preferably 30 cSt or more, preferably 40 cSt or more, preferably 100 or more, preferably 150 cSt or more). In one or more embodiments, the PAO has a KV100°C of 3 to 3,000 cSt (preferably 4 to 1,000 cSt, preferably 6 to 300 cSt, preferably 8 to 150 cSt, preferably 8 to 100 cSt, preferably 8 to 40 cSt). In one or more embodiments, the PAO or blend of PAOs has a KV100°C of 10 to 1000 cSt (preferably 10 to 300 cSt, preferably 10 to 100 cSt). In yet another embodiment, the PAO or blend of PAOs has a KV100°C of 4 to 8 cSt. In yet another embodiment, the PAO or blend of PAOs has a KV100°C of 25 to 300 cSt (preferably 40 to 300 cSt, preferably 40 to 150 cSt). In one or more embodiments, the PAO or blend of PAOs has a KV100°C of 100 to 300 cSt.

In one or more embodiments, the PAO or blend of PAOs has a Viscosity Index (VI) of 120 or more (preferably 130 or more, preferably 140 or more, preferably 150 or more, preferably 170 or more, preferably 190 or more, preferably 200 or more, preferably 250 or more, preferably 300 or more). In one or more embodiments, the PAO or blend of PAOs has a VI of 120 to 350 (preferably 130 to 250).

In one or more embodiments, the PAO or blend of PAOs has a pour point of -10°C or less (preferably -20°C or less, preferably -25°C or less, preferably -30°C or less, preferably -35°C or less, preferably -40°C or less, preferably -50°C or less). In one or more embodiments, the PAO or blend of PAOs has a pour point of -15 to -70°C (preferably -25 to -60°C).

In one or more embodiments, the PAO or blend of PAOs has a glass transition temperature (T_{g}) of -40°C or less (preferably -50°C or less, preferably -60°C or less, preferably -70°C or less, preferably -80°C or less). In one or more embodiments, the PAO or blend of PAOs has a T_{g} of -50 to -120°C (preferably -60 to -100°C, preferably -70 to -90°C).

In one or more embodiments, the PAO or blend of PAOs has a flash point of 200°C or more (preferably 210°C or more, preferably 220°C or more, preferably 230°C or more), preferably between 240°C and 290°C. In one or more embodiments, the PAO or blend of PAOs has a specific gravity (15.6°C) of 0.86 or less (preferably 0.855 or less, preferably 0.85 or less, preferably 0.84 or less).

In one or more embodiments, the PAO or blend of PAOs has a molecular weight distribution (M_{w}/Mₙ) of 2 or more (preferably 2.5 or more, preferably 3 or more, preferably 4 or more, preferably 5 or more, preferably 6 or more, preferably 8 or more, preferably 10 or more). In one or more embodiments, the PAO or blend of PAOs has a M_{w}/Mₙ of 5 or less (preferably 4 or less, preferably 3 or less) and a KV100°C of 10 cSt or more (preferably 20 cSt or more, preferably 40 cSt or more, preferably 60 cSt or more).

Desirable PAOs are commercially available as SpectraSyn™ and SpectraSyn ULtra™ from ExxonMobil Chemical (USA). Other useful PAOs include those available as Synfluid™ from ChevronPhillips Chemical (USA), as Durasyn™ from Innovene (USA), as Nexbase™ from Neste Oil (Finland), and as Synton™ from Chemtura (USA). For PAOs, the percentage of carbons in chain-type paraffinic structures (C_{P}) is close to 100% (typically greater than 98% or even 99%). Additional details are described in, for example, U.S. Patent No(s). 3,149,178; 4,827,064; 4,827,073; 5,171,908; and 5,783,531; and in Synthetic Lubricants and High-Performance Functional Fluids (Leslie R. Rudnick & Ronald L. Shubkin, ed. Marcel Dekker, Inc. 1999), pp. 3-52.

### Additives

Any of the resins or layers can further include one or more additives. Suitable additives can include any one or more processing oils (aromatic, paraffinic and napthathenic mineral oils); compatibilizers; calcined clay; kaolin clay; nanoclay; talc; silicates; carbonates; sulfates; carbon black; sand; glass beads; mineral aggregates; wollastonite; mica; glass fiber; other filler; pigments; colorants; dyes; carbon black; filler; dispersants; flame retardants; antioxidants; conductive particles; UV-inhibitors; stabilizers; light stabilizer; light absorber; coupling agents including silanes and titanates; plasticizers; lubricants; blocking agents; antiblocking agents; antistatic agents; waxes; foaming agents; nucleating agents; slip agents; acid scavengers; lubricants; adjuvants; surfactants; crystallization aids; polymeric additives; defoamers; preservatives; thickeners; rheology; modifiers; humectants; coagents; vulcanizing/cross-linking/curative agents; vulcanizing/cross-linking/curative accelerators; cure retarders, and combinations thereof.

### Process for making composite

The multilayer composite or fabric can be formed using any melt blowing process. Preferably, the multilayer composite is meltblown from an apparatus that can operate at a melt pressure from greater than 500 psi (3.45 MPa) and a melt temperature within the range of 100°C to 350°C and capable of making fibers as fine as 1 micron in average diameter.

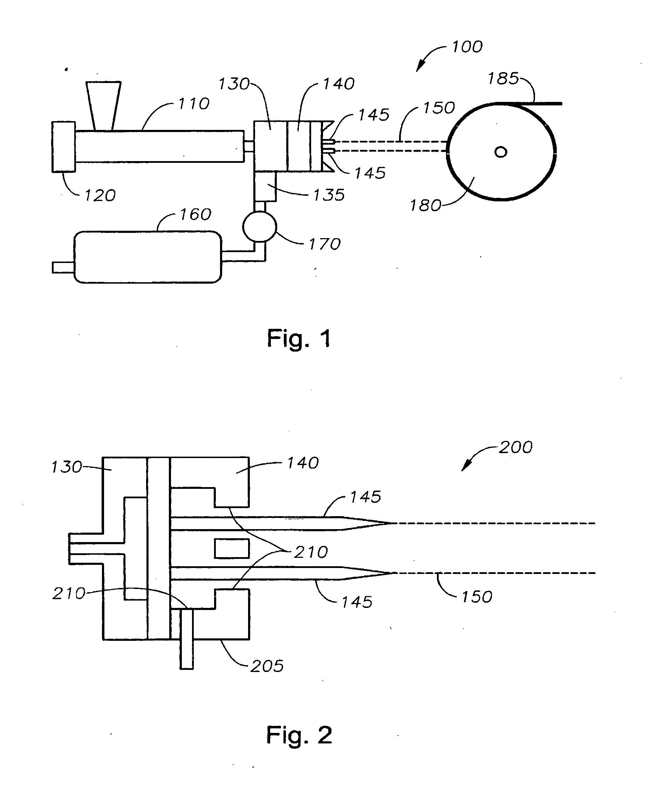

Figure 1 depicts a schematic view of an illustrative meltblowing system or arrangement 100 for making the multilayer meltblown composite, according to one or more embodiments. The system 100 includes at least one extruder 110, and may include a motor 120 to maintain melt pressure within the system 100. The extruder 110 can be coupled to at least one die block or array die 130 that is coupled to a spinneret portion or spinneret 140. The die block 130 is also coupled to at least one air manifold 135 for delivering high pressure air to the spinneret portion 140 of the die block 130. The spinneret 140 includes a plurality of spinning nozzles 145 through which the melt is extruded and simultaneously attenuated with air pressure to form filaments, or fibers 150. The spinning nozzles 145 are preferably circular, die capillaries. Preferably, the spinneret 140 has a nozzle density that ranges from 20, 30, or 40 holes/inch (holes/2.54cm) to 200, 250, or 320 holes/inch (holes/2.54cm). In one embodiment, each nozzle 145 has an inside diameter ranging of from about 0.05 mm, 0.10 mm, or 0.20 mm to 0.80 mm, 0.90 mm, or 1.00 mm.

In the die spinneret 140, the molten threads or filaments converge with a hot, high velocity, gas stream (e.g., air or nitrogen) to attenuate the filaments of molten thermoplastic material to form the individual fibers 150. The temperature and flow rate of the attenuating gas stream can be controlled using a heat exchanger 160 and air valve 170. The diameters of the filaments can be reduced by the gas stream to a desired size. Thereafter, the meltblown fibers 150 are carried by the high velocity gas stream and are deposited on a collecting surface 180 to form at least one web 185 of randomly disbursed meltblown fibers. The collecting surface 180 can be an exterior surface of a vacuum drum, for example.

Figure 2 depicts an enlarged schematic view of an illustrative die assembly 200, according to one or more embodiments. The die assembly 200 includes the die block 130 and the spinneret 140. As depicted, the air ("primary air") is provided through the primary air nozzle 210 located at least on a side of the die spinneret 140. The die block 130 can be heated using the primary air, a resistive heating element, or other known device or technique (not shown), to prevent the die block 130 from becoming clogged with solidifying polymer as the molten polymer exits and cools. The air also draws, or attenuates, the melt into fibers. Secondary, or quenching, air at temperatures above ambient can also be provided through the die block 130. Primary air flow rates typically range from about 1 to 30 standard cubic feet per minute per inch of die width (SCFM/inch) (SCFM/2.54 cm). In certain embodiments, the primary air pressure in the meltblown process typically ranges from a low of about 2 psig (14 kPa), 3, psig (21 kPa), 5 psig (34 kPa), or 7 psig (48 kPa) to about 10 psig (69 kPa), 15 psig (103 kPa), 20 psig (138 kPa), or 30 psig (207 kPa) at a point in the die block 130 just prior to exit. Primary air temperatures are typically within the range from 150°C, 200°C, or 230°C to 300°C, 320°C, or 350°C.

The melting temperature (Tm) of the resins can range from 50°C to 300°C. In yet other embodiments, the melting temperature is at least 50°C and less than 150°C; 200°C; 220°C; 230°C; 250°C; 260°C; 270°C; 280°C; 290°C; 300°C; 310°C; or 320°C. The resin can be formed into fibers at a melt pressure from greater than 500 psi (3.4 MPa); or 750 psi (5.2 MPa); or 1,000 psi (6.9 MPa); or 2,000 psi (17.3 MPa); or within the range from 500 psi (3.5 MPa); or 750 psi (5.2 MPa); to 1,000 psi (6.9 MPa); or 2,000 psi (13.8 MPa); or 2,500 psi (17.3 MPa).

Expressed in terms of the amount of composition flowing per inch of the die per unit of time, throughputs for the manufacture of meltblown fabrics using the compositions described herein are typically within the range from 0.1; 0.2; 0.3; or 0.5 to 1.0; 1.5; 2.0; or 3.0 grams per hole per minute (ghm). Thus, for a die having 30 holes per inch (2.54 cm), polymer throughput is typically about 0.25, 0.5, or 1.0 to 4, 8, or 12 lbs/inch/hour (PIH) (0.045, 0.089, 0.179 to 0.715, 1.43 or 2.145 kg/cm/h).

Because such high temperatures can be used, a substantial amount of heat is desirably removed from the fibers in order to quench, or solidify, the fibers leaving the nozzles. Although not shown, cold gases of air or nitrogen can be used to accelerate cooling and solidification of the meltblown fibers. In particular, cooling ("secondary") air flowing in a cross-flow direction (perpendicular or angled) relative to the direction of fiber elongation may be used to quench meltblown fibers. Also, an additional, cooler pressurized quench air may be used and can result in even faster cooling and solidification of the fibers. In certain embodiments, the secondary cold air flow may be used to attenuate the fibers. Through the control of air and array die temperatures, air pressure, and polymer feed rate, the diameter of the fiber formed during the meltblown process may be regulated.

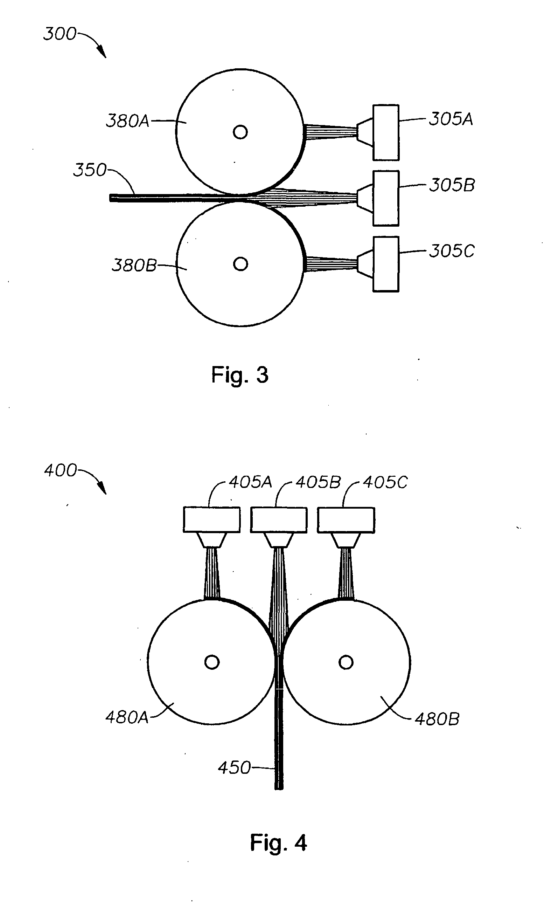

Figure 3 depicts a schematic of an illustrative meltblowing system 300 for making a multilayer meltblown laminate or composite 350, according to one or more embodiments. The meltblowing system 300 can include three or more vertically arranged dies 305A, 305B, 305C. Each die 305A, 305B, 305C can be similar to the die 200 discussed and described above with reference to Figure 2. Any resin or combination of resins can be blown through any given die 305A, 305B, 305C, where the first die 305A provides a first facing or first outer layer, the second die 305B provides a core layer or intermediate layer, and the third die 305C provides a second facing layer or second outer layer.

The meltblowing system 300 can further include two or more collection surfaces 380A, 380B that are vertically aligned. Each collection surface 380A, 380B can be similar to the collection drum 180 depicted and described above with reference to Figure 1. The collection surfaces 380A, 380B can be adjacent one another such that a desired gap ("nip") is defined therebetween. As depicted, fibers from each die 305A, 305B, 305C are horizontally directed toward and collected on the collection surfaces 380A and/or 380B to form a three layer fabric composite 350. The dies 305A, 305B, 305C can be independently movable with respect to one another. The dies 305A, 305B, 305C can also be independently movable with respect to the collection surfaces 380A, 380B to vary the die to collector distance ("DCD").

Figure 4 depicts a schematic of another illustrative meltblowing system 400 for making a multilayer meltblown laminate or composite 450, according to one or more embodiments. The meltblowing system 400 can include three or more horizontally arranged dies 405A, 405B, 405C and horizontally aligned collection surfaces 480A, 480B. Each die 405A, 405B, 405C can be similar to the die 200 discussed and described above with reference to Figure 2. Each collection surface 480A, 480B can be similar to the collection drum 180, as depicted and described above with reference to Figure 1. The dies 405A, 405B, 405C can be independently movable with respect to one another. The dies 405A, 405B, 405C can also be independently movable with respect to the collection surfaces 480A, 480B to vary the DCD.

Any resin or combination of the resins can be vertically extruded through any given die 405A, 405B, 405C to provide a multi-layer composite having first and second facing layers disposed about a core layer, as described herein. As depicted, fibers from each die 405A, 405B, 405C are directed toward and collected on the collection surfaces 480A and/or 480B to form a three layer fabric composite 450.

Referring to any system or arrangement described above 100, 200, 300, 400, the laminate may be passed through the nip between the unheated or heated smooth collection surface(s), or unheated or heated patterned collection surface(s), or a combination of two or more of these, while applying light pressure thereon, as another extensible construction is contacted with the laminate to form a multilayer construction. Given the formation of the multilayer constructions as described herein, in certain embodiments, adhesives are substantially absent from the constructions, meaning that adhesives are not used to secure the layers of fabric and/or film to one another. As used herein, an "adhesive" is a substance that is used to secure two layers of film or fabric to one another as is known in the art. Examples of adhesive substances include polyolefins, polyvinyl acetate polyamides, hydrocarbon resins, waxes, natural asphalts, styrenic rubbers, and blends thereof.

The meltblown fibers may be continuous or discontinuous and are generally within the range from 0.5 to 250 µm in average diameter, preferably less than 200 µm, less than 150 µm, less than 100 µm, less than 75 µm, less than 50 µm, less than 40 µm, less than 30 µm, less than 20 µm, less than 10 µm, less than 5 µm, less than 4 µm, less than 3 µm, less than 2 µm, or less than 1 µm. In certain embodiments, the meltblown fibers can have a diameter within the range of from 5; or 6; or 8; or 10 to 20; or 50; or 80; or 100; or 150; or 200; or 250 µm in average diameter, and in other embodiments have a diameter from less than 80 or 50 or 40 or 30 or 20 or 10 or 5 µm.

The fiber diameters of each layer of the multi-layered composite can be the same or different. Accordingly, a ratio of fiber diameters of adjacent layers can be the same or vary. For example, a ratio of fibers diameters of adjacent layers can range from a low of 0.1:1 to a high of 1:200. Such ratios can also range from 1:150; 1:100; 1:75; 1:50; 1:25; 1:10; 1:5; or 1:2.

At least 1% of the fibers in any given layer of the multi-layered structure can be co-joined or married. More preferably, at least 2%; 5%; 10%; 15%; 20%; or 25% of the fibers in any given layer of the multi-layered structure can be co-joined or married. The amount of co-joined or married fibers can also range from a low of 1%; 5%; or 10% to a high of 25%; 35%; or 45%.

The fibers of any one or more layers of the multi-layered structure can exhibit or possess some extent of fusion, melting, entrainment or mechanical interlocking with the fibers of any one or more adjoining layers without a sharp delineated interface between layers.

At least one layer of the multi-layered structure can recover at least 80% of its original length after 100% extension and at least 70% of its original length after 200% extension. In one or more embodiments, the multi-layered structure can recover at least 80% of it original length after 100% extension and at least 70% of its original length after 200% extension.

The force at 50% extension of at least one layer of the multi- layered structure, upon elongating the sample to 100% of its original length and then upon unloading, is 1.3x10⁻³ lbf/in/gsm (0.23 kg/cm/gsm).

The multi-layered structure has a hydrohead of about 0.05 mbar/gsm or more. Preferably, the hydrohead is greater than 0.1 mbar/gsm (10 Pa/gsm), 0.2 mbar/gsm (20 Pa/gsm), 0.3 mbar/gsm (30 Pa/gsm), 0.4 mbar/gsm (40 Pa/gsm), or 0.5 mbar/gsm (50 Pa/gsm). The hydrohead can also range from a low of 0.1 mbar/gsm (10 Pa/gsm), 0.2 mbar/gsm (20 Pa/gsm) or 0.3 mbar/gsm (30 Pa/gsm) to a high of 0.7 mbar/gsm (70 Pa/gsm), 0.8 mbar/gsm (80 Pa/gsm), or 0.9 mbar/gsm (90 Pa/gsm).

The air permeability of any one or more layers of the multi-layered structure is 0.02 cm³/cm²/s or more. In one or more embodiments, the air permeability of the multi-layered structure is about 0.02 cm³/cm²/s or more. The air permeability can also range from a low of 0.02 cm³/cm²/s, 0.05 cm³/cm²/s, or 1.0 cm³/cm²/s to a high of 2.0 cm³/cm²/s, 3.0 cm³/cm²/s or 5.0 cm²/cm²/s.

The fabrics may have a basis weight within the range of from 10 or 20 or 30 to 50 or 80 or 100 or 150 g/m². These fabrics may also be characterized by having an Ultimate Elongation from greater than 200% or 300% or 500% or 1,000%. In this manner, multilayer constructions can be formed having at least three melt-blown layers ("MMM"). Other multi-layered meltblown structures are contemplated such as MₓQ; QMₓQ; Mₓ; QMₓ; QₓS; MₓA_{y}M_{y}; QMₓA_{y}M_{y}Q; QMₓQM_{y}S; QMₓQM_{y}; MₓQM_{y}Q; QQMₓQ, where x is at least 3 and y is 0 to 100. For example, x can be 3 to 100; 3 to 50; 3 to 25; or 3 to 10; x can also range from a low of about 3, 4, or 5 to a high of about 6, 10, or 15; x can also range from a low of about 1, 2, 3, 4, or 5 to a high of about 6, 7, 8, 10, or 15. "M" represents a layer of meltblown fabric (where each "M" in a construction may be the same or different); "Q" represents a spunbond, spunlace, woven fabric, or film (where each "S" in a construction may be the same or different), and "A" represents one or more additives. When such adhering of the meltblown fibers to another fabric is desired, the secondary cooling air flow may be diminished and/or heated to maintain some of the melt quality and hence bonding ability of the forming elastic meltblown fibers to the fabrics upon which they are bonded.

More particularly, in forming a multilayered construction, the polyolefin polymers may be meltblown onto an extensible fabric, such as a spunlace fabric, that is passed underneath or in front of the forming meltblown fabric. The melt temperature and distance between the spinnerets and the passing extensible fabric is adjusted such that the fibers are still in a melt or partial melt state when contacting the fabric(s) to form a two or three layer construction. The coated fabric(s) then has the melted or partially-melted elastic meltblown fibers/fabric adhered thereto.

The multilayered construction can then be mechanically stretched to tailor the elastic performance of the composite. Not wishing to be bound by theory, it is believed that initial stretching modifies the structure of the elastomeric components in the composite, and potentially the interfacial bonding among fibers between and/or within layers. An initial stretching can reduce the hysteresis loop, which is a measure of the energy absorbed by the elastomer during deformation. An ideal elastomer has no hysteresis, or put another way, all the energy put into the elastomer, or stored in the elastomers, is given back upon returning the elastomer to its original size and shape. There are few elastomers and even fewer elastic laminates that show ideal elastic behavior. Most if not all show some level of hysteresis. An initial loading and unloading cycle will typically reduce the hysteresis loop which means that the material or laminate is a more efficient elastomer. The mechanical stretching of elastomers and elastomeric composites can have other advantageous effects, such as reducing the peak load at deformation, potentially improved permanent set, retractive force, and adjusting the aesthetics of the outer layers/surfaces.

There are many different methods for mechanically stretching a composite in both machine direction (MD) and cross-direction (CD). Devices based upon intermeshing blades or disks are effective at incrementally stretching fabrics in either MD or CD, respectively, or both when units are placed in series. The term incremental stretching arises from the fact the fabrics are stretched in an incremental fashion across their entire width or length. The increment or distance over which the fabric is stretched is determined by the spacing of adjacent disks or blades and the distance of interpenetration between the two sets of disks or blades. Examples of this and similar technology using grooved rolls rather than separate disks can be found in U.S. Patent No(s). 4,223,059; 4,251,585; 4,285,100; and 4,368,565. Further improvements to this basic technology allowing narrower webs/films to be efficiently stretched, or to increase the amount of stretching or vary the amount of stretch across a web can be found in U.S. Patent No(s). 5,143,679; 5,156,793; and 5,167,897.

Other technologies are available for stretching webs that are better suited for MD stretching. An example of using nip rolls for this purpose is described in U.S. Patent No. 7,320,948 in which sets of two nip rolls running at different speeds enable fabrics and laminates to be stretched in MD.

### Fiber

The fiber can be a single component fiber. The fiber can also be a multi-component fiber formed from a process wherein at least two polymers are extruded from separate extruders and melt-blown or spun together to form one fiber. In one or more embodiments, the polymers used in the multi-component fiber are the same or substantially the same. In one or more embodiments, the polymers used in the multi-component fiber are different from each other. The configuration of the multi-component fiber can be, for example, a sheath/core arrangement, a side-by-side arrangement, a pie arrangement, an islands-in-the-sea arrangement, or a variation thereof. The fiber can also be drawn to enhance mechanical properties via orientation, and subsequently annealed at elevated temperatures, but below the crystalline melting point to reduce shrinkage and improve dimensional stability at elevated temperature.

Where a separate fabric or layer is unwound into the process, and is for example, used as a facing layer for the laminate, these fabrics can be continuous fibers such as found in spunbonded fabrics, staple fibers, or discontinuous fibers, such as those found in carded fabrics. The length and diameter of the staple fibers can vary depending on the desired toughness and stiffness of the fiber reinforced composition. In one or more embodiments, the fibers have a length of 1/4 inch, (0.635 cm), or a length within the range having a lower limit of 1/8 inch (0.3175 cm), or 1/6 inch (0.423 cm), and an upper limit of 1 inch (2.54 cm), or 1.5 inch (3.81 cm) or 5 inch (12.70 cm). In one or more embodiments, the diameter of the fibers is within the range having a lower limit of 0.1 µm and an upper limit of 100 µm. The diameters can also range from a low of 0.1 µm, 0.5 µm, or 1.0 µm to a high of about 5 µm, 10 µm or 15 µm. Suitable ranges also include 0.1 to 8 µm; 0.2 to 7 µm; 0.3 to 6 µm, 0.1 of 5 µm; and 0.1 to 3 µm.

The mechanical properties of the meltblown fabrics (or multilayer constructions) described herein can be enhanced by a stretching or orientation process. Annealing can be combined with mechanical orientation, in either or both the CD or the MD. If desired, mechanical orientation can be done by the temporary, forced extension of the fabric for a short period of time before it is allowed to relax in the absence of the extensional forces. In the meltblowing process, there may be some degree of orientation of the fibers in the MD imparted due to the laydown or spinning process alone. But in certain embodiments, no additional mechanical orientation or stretching is needed. Thus, in certain embodiments, the meltblown fabrics described herein have a low degree of, or no, orientation. In other embodiments, orientation is imparted in the CD but not the MD. Thus, in certain embodiments, the meltblown fabric possesses an MD Elongation less than 20 or 50 or 80 or 100% and a CD Elongation greater than 100 or 200 or 300%. Stated another way, the meltblown fabric possesses a CD/MD elongation at break ratio of between 0.1 or 0.5 and 2 or 3 or 5 or 7 or 10.

The formation of the elastic fibers and fabrics includes an annealing step with or without mechanical orientation. Annealing may also be done after fabrication of the fabric from the elastic fibers. In certain embodiments, the elastic meltblown fiber or fabric is annealed at a temperature within the range from 50°C or 60°C to 130°C or 160°C. Thermal annealing of the fabric is conducted by maintaining the fabric at a temperature within the range above for a period from 1 second to 1 minute, preferably between 1 and 10 seconds. The annealing time and temperature can be adjusted for any particular copolymer or copolymer composition. In another embodiment, the meltblown fabrics can be annealed in a single-step by a heated roll during calendaring under low tension. In other embodiments, the meltblown fabrics require little to no post fabrication processing.

The forming multilayer construction is further processed by passing the multilayer construction through a hydroentangling apparatus, thus further bonding the web of elastic fibers to each other or other adjacent fabric layers by interlocking and entangling the fibers about each other with high velocity streams of water. Hydroentangling is known in the art and described in some detail by A.M. Seyam et al., "An Examination of the Hydroentangling Process Variables," INT'L NONWOVENS J., pp. 25-33 (Spring 2005).

As mentioned above, the fibers can be continuous (long fibers) or discontinuous (short fibers). Long fibers will have a length to diameter aspect ratio greater than 60, preferably 200 to 500; and the short fibers will have a length to diameter aspect ratio less than 60, preferably 20 to 60_{.} The number of fibers per square inch (fiber,density) of the meltblown fabric preferably ranges from a low of 20 fibers/in² (3 fibers/cm²), 40 fibers/in² (6 fibers/cm²), or 50 fibers/in² (8 fibers/cm²) to a high of 100 fibers/in² (15 fibers/cm²), 250 fibers/in² (39 fibers/cm²), or 500 fibers/in² (77 fibers/cm²). Suitable ranges also include: 25 fibers/in² (4 fibers/cm²) to 400 fibers/in² (62 fibers/cm²); 50 fibers/in² (8 fibers/cm²) to 300 fibers/in² (47 fibers/cm²); 60 fibers/in² (9 fibers/cm²) to 200 fibers/in² (31 fibers/cm²); 20 fibers/in² (3 fibers/cm²) to 80 fibers/in² (12 fibers/cm²); and 30 fibers/in² (7 fibers/cm²) to 70 fibers/in² (11 fibers/cm²).

### Specific Layer Blends

In one or more preferred embodiments, at least one layer or fabric of the multilayer composite can include at least one propylene-α-olefin copolymer ("PCP"). The at least one layer can optionally include one or more polypropylenes. For example, the at least one layer can contain 50 wt% of one or more propylene-α-olefin copolymers and 50 wt% of one or more polypropylene resins. The amount of the propylene-α-olefin copolymer resin in the layer can be at least 5 wt%; 10 wt%; 20 wt%; 30 wt%; 40 wt%; 50 wt%; 60 wt%; 70 wt%; 80 wt%; 85 wt%; 90 wt%; 95 wt%; 97 wt%; 98 wt%; 99 wt%; or 100 wt%. The layer range can consist essentially of the propylene-α-olefin copolymer resin. The amount of the propylene-α-olefin copolymer resin in the layer can also range of from a low of 40 wt%, 50 wt%, or 60 wt% to a high of 75 wt%, 85 wt%, or 100 wt%. The amount of the polypropylene resin in the layer can range from a low of 1 wt%; 5 wt%; or 10 wt% to a high of 20 wt%; 40 wt%; or 60 wt%. The preferred PCP has an ethylene content of 12 wt% to 20 wt%; or about 13 wt% to 16 wt%; or 14 to 15 wt%; or 15 wt%. The preferred PCP further has an MFR (ASTM-1238D, 2.16 kg, 230°C) of 10 dg/min to 30 dg/min; 12 dg/min to 25 dg/min; or 14 dg/min to 23 dg/min or 16 dg/min to 21 dg/min; or 18 dg/min to 19 dg/min; or 18 dg/min. The preferred PCP has a heat of fusion (H_{f}) of 75 J/g or less; 70 J/g or less; 65 J/g or less; 60 J/g or less; or 57 J/g or less, and H_{f} can range from a low of 30; 35; or 40 J/g, to a high of 55; 65; or 75 J/g.

In one or more preferred embodiments, at least one layer of the multilayer composite includes at least one propylene-based or ethylene-based homopolymers or random, block, or graft copolymers comprising none (i.e. homopolymers) or from 0.1 wt%; or 1 wt%; or 2 wt%; or 5 wt% to 10 wt%; or 15 wt%; or 20 wt%; or 45 wt%, of the polymer, of comonomer-derived units selected from ethylene and C₄-C₁₀ α-olefins (propylene-based polymers) and C₃-C₁₀ α-olefins (ethylene-based polymers). Preferably, at least one layer of the multilayer composite includes one or more polypropylenes within the range of from 50 wt% to 99 wt%; or 60 wt% to 95 wt%; or 50 wt% to 90 wt%; or 55 wt% to 85 wt%, of the fabric layer/composition. In one or more embodiments, at least one layer of the multilayer composite consists essentially of one or more polypropylenes.

In one or more preferred embodiments, at least one layer or fabric of the multilayer composite includes a blend of polypropylene and less than 50 wt% of one or more blend components. The blend component can be one or more impact copolymers, one or more random copolymers (RCP), one or more polyethylenes, one or more polyethylenes having an Mw of less than 20,000 g/mol, one or more polypropylenes having a Mw of less than 20,000 g/mol, one or more polyalphaolefins, or any combination(s) thereof. The amount of the blend component (not the polypropylene) can be present in an amount ranging from a low of 0.5 wt%; 1 wt%; or 5 wt% to a high of 30 wt%; 40 wt%; or 50 wt%. For example, the amount of the blend component can be of from 1 wt% to 49 wt%; or 5 wt% to 45 wt%; or 5 wt% to 40 wt%; or 5 wt% to 25 wt%.

A preferred multilayer composite has at least one facing layer wherein the MFR (ASTM D1238, 230°C, 2.16 kg) of the facing layer resin or blend is less than 2,000 dg/min (g/10 min); preferably 1,500 dg/min or less; 1,200 dg/min or less; 900 dg/min or less; 600 dg/min or less; 300 dg/min or less; 200 dg/min or less; 150 dg/min or less; 100 dg/min or less; or 90 dg/min or less. In certain embodiments, the MFR of the extensible resin or blend can range from a low of about 50 dg/min; 75 dg/min; or 80 dg/min, to a high of about 250 dg/min; 500 dg/min; or 1,000 dg/min. The MFR of the facing layer resin or blend can also range from a low of about 20 dg/min; 30 dg/min; or 40 dg/min, to a high of 90 dg/min; 120 dg/min; or 150 dg/min. The MFR of the facing layer resin or blend can also range from a low of 20 dg/min; 35 dg/min; or 45 dg/min, to a high of about 65 dg/min; 80 dg/min; or 95 dg/min. The MFR of the facing layer resin or blend can further range from a low of 0.1 dg/min; 0.5 dg/min; 1 dg/min or 5 dg/min to a high of about 30 dg/min; 40 dg/min; 70 dg/min; or 90 dg/min.

The weight average molecular weight (Mw) of the facing layer resin or blend is preferably less than 500,000; 400,000; 300,000; or 250,000. For example, the Mw of the facing layer resin or blend can range from about 50,000, to about 200,000. In one or more embodiments, the Mw of the facing layer resin or blend can range from a low of 50,000; 80,000; or 100,000, to a high of 155,0001 170,0001 or 190,000. In one or more embodiments, the Mw of the facing layer resin or blend can range from 80,000 to 200,000; 100,000, to about 175,000; or 140,000, to 180,000.

A preferred multilayer composite also has at least one core layer wherein the MFR (ASTM D1238, 230°C, 2.16 kg) of the core layer resin or blend is preferably less than 2,000 dg/min (g/10 min); more preferably 1,500 dg/min or less; 1,200 dg/min or less; 900 dg/min or less; 600 dg/min or less; 300 dg/min or less; 200 dg/min or less; 150 dg/min or less; 100 dg/min or less; or 90 dg/min or less. In certain embodiments, the MFR of the core layer resin or blend can range from a low of about 50 dg/min; 75 dg/min; or 80 dg/min, to a high of 250 dg/min; 500 dg/min; or 1,000 dg/min. The MFR of the core layer resin or blend can also range from a low of 20 dg/min; 30 dg/min; or 40 dg/min, to a high of 90 dg/min; 120 dg/min; or 150 dg/min. The MFR of the core layer resin or blend can also range from a low of 25 dg/min; 35 dg/min; or 45 dg/min, to a high of 75 dg/min; 85 dg/min; or 95 dg/min. The MFR of the core layer resin or blend can further range from a low of 0.1 dg/min; 0.5 dg/min; 1 dg/min; or 5 dg/min, to a high of about 30 dg/min; 40 dg/min; 70 dg/min; or 90 dg/min. In at least one specific embodiment, the MFR of the core layer resin or blend ranges from 2 dg/min to 90 dg/min; 2 dg/min to 20 dg/min; 3 dg/min to 90 dg/min; or 3 dg/min to 20 dg/min.

The weight average molecular weight (Mw) of the core layer resin or blend is preferably less than 500,000; 400,000; 300,000; or 250,000. For example, the Mw of the core layer resin or blend can range from 50,000 to 290,000. In one or more embodiments, the Mw of the core layer resin or blend can range from a low of 50,000; 65,000; or 80,000, to a high of 130,000; 190,000; or 290,000. In one or more embodiments, the Mw of the core layer resin or blend can range from 80,000 to 285,000; 80,000, to 240,000; or 80,000 to 140,000.

One method of characterizing multilayer construct elasticity is to determine a hysteresis curve according to the following cyclic testing procedure. Generally, a sample of nonwoven fabric is stretched one or more times using an Instron 1130 instrument, which is commercially available from Instron Corporation. Unless stated otherwise, the test parameters used herein to generate hysteresis curves are: sample width = 1 inch (2.54 cm), sample length = 3 inches (7.6 cm), gauge length, i.e., distance between clamps, is 1 inch (2.54 cm), crosshead speed, i.e., speed of top clamp that is applying a stretching force, is 10 in/min (25.4 cm/min). As used herein "first cycle" and "second cycle" refer to the number of times an individual sample has been stretched.

Samples are tested by first cutting a nonwoven fabric sample to the specified sample size. Each test sample is loaded in to an Instron 1130 instrument by first attaching the sample to the crosshead/top clamp and then to the bottom clamp. The distance between the clamps is the specified gauge length. No pre tension is applied on the sample.

The sample is then stretched to the desired strain, e.g., 100%, or 200%, as measured by sample length, using a crosshead speed, i.e., stretch speed, of 10 in/min (2.54 cm/min). The sample is then returned to zero load at the same crosshead speed without any hold time. The force on the sample as a function of strain during extension and retraction is recorded.

The sample is removed from the instrument for further characterization or stretched one or more times if additional cycles data was desired, e.g., second cycle data. Second cycle hysteresis curves are prepared by remounting samples already tested in a first cycle. Samples are mounted using the same gauge length unless specifically reported otherwise. The same procedure described above for the first cycle is utilized for the second cycle.

Unless described otherwise herein, permanent set is the amount of strain remaining in a sample after retraction from a specified strain expressed as a percentage of the specified strain. The elongation remaining in the sample at zero load after retraction (as determined by the intercept of the retraction curve with the x-axis) is divided by the maximum elongation the sample was stretched during that cycle.

Unless described otherwise herein, retractive force at 50% is the force exerted by a sample after stretching to a given elongation and allowing the sample to retract to one-half of that elongation.

Unless described otherwise herein, peak load (lbs/in) (0.454 kg/2.54 cm) is the maximum load in pounds force (kg force) exerted on the sample during extension divided by the width of the sample in inches (2.54 cm).

Unless described otherwise herein, peak force MD (N) is the maxium force exerted on a sample during extension in the machine direction (MD) expressed in Newtons.

Unless described otherwise herein, peak force CD (N) is the maximum force exerted on a sample during extension in the cross direction (CD) expressed in Newtons.

Unless described otherwise herein, elongation at break MD (%) is the increase in length of a sample measured at the breaking point after extension in the machine direction divided by the original gauge length expressed as a percentage.

Unless described otherwise herein, elongation at break CD (%) is the increase in length of a sample measured at its breaking point after stretching in the cross direction divided by the original gauge length expressed as a percentage.

### Articles

The multilayer constructions are particularly useful for applications requiring any one or more of the following properties or attributes: absorbency, liquid repellency, resilience, stretch, softness, strength, flame retardancy, washability, cushioning, filtering, bacterial barrier, and sterility. Illustrative applications and uses can include, but are not limited to, hygiene, medical, filters, and geotextiles, among others.

For example, the multilayer constructions can be used to make baby diapers, feminine hygiene napkins, adult incontinence products, personal hygiene wipes, bandages, wound dressings, air filters, liquid filters, household wipes, shop towels, battery separators, vacuum cleaner bags, cosmetic pads, food packaging, clothing, apparels, medical garments, and disposable underwear. Particularly suitable uses include closure systems on baby diapers, pull-ups, training pants, adult incontinence briefs and diapers, bandages, and other single use or disposable items.

Common filtering uses include gasoline, oil, and air filters; water, coffee and tea bags; liquid cartridge and bag filters; vacuum bags; and allergen membranes. Illustrative geotextiles and uses thereof include soil stabilizers and roadway underlayment, foundation stabilizers, erosion control, canals construction, drainage systems, geomembranes protection, frost protection, agriculture mulch, pond and canal water barriers, and sand infiltration barrier for drainage tile.

Additional articles and uses of the multilayer construction provided herein can include, for example, carpet backing, marine sail laminates, table cover laminates, chopped strand mat, backing/stabilizer for machine embroidery, packaging, insulation, pillows, cushions, and upholstery padding, batting in quilts or comforters, consumer and mailing envelopes, tarps, as well as tenting and transportation (lumber, steel) wrapping.

The entire article can be formed from the multiplayer constructions, or the multilayer constructions can form individual sections or portions thereof. For example, in baby diapers, it is envisaged that the multilayer constructions form at least part of the back sheet, wings, and/or tabs.

### EXAMPLES

The foregoing discussion can be further described with reference to the following non-limiting examples. In the examples provided, meltblown fabrics and multilayer constructions were formed using equipment and conditions similar to that of R. Zhao, "Melt Blowing Polyoxymethylene Copolymer," INT'L NONWOVENS J., pp. 19-24 (Summer 2005). In particular, a Biax-Fiberfilm™ meltblown line (Biax-Fiberfilm Corp., Greenville, WI) operating at a melt pressure within the range from 1200 psi (6.89 MPa) to 1700 psi (10.34 MPa) and a melt temperature within the range from 200°C to 275°C, using an array die with a spinneret hole density of between 50 and 150 holes/inch (holes/2.54 cm) was used to form the meltblown fabrics and multilayer constructions. The line had an extruder, die-block, and spinneret, as well as an air manifold for the spinneret supplying air pressures within the range from 5 psi to 20 psi (34 kPa to 138 kPa) and air temperatures within the range from 220°C to 260°C.

In each example, a propylene-α-olefin copolymer ("PCP-01") possessing an MFR of 18 dg/min and ethylene content of 15 wt% was meltblown under these conditions using the Biax-Fiberfilm™ meltblown line to form the fabrics and multilayer construction. The PCP-01 was melt blended in the extruder, and meltblown via the Biax-Fiberfilm™ array die, onto an extensible construction of spunlace fabric that was passed underneath or in front of the forming fibers of the meltblown PCP-01. The fiber average diameter was within the range of from 15 µm to 45 µm. The melt temperature and distance between the spinnerets and the passing spunlace fabric was adjusted such that the fibers were still in a melt or partial melt state when contacting the spunlace fabric(s) to form a two or three layer construction.

Table 1 shows the data for samples 1 through 4 that were each produced using a 15" (38.1 cm) die with 0.020" (0.508 mm) nozzles arrayed in two holes at low throughput, about 0.15 ghm. The facing layer was a spunlaced 30 gsm 50/50 blend of PP and PET staple fibers.

Table 2 shows the data for samples 5 through 8 that were each produced using a 15" (38.1 cm) die with 0.020" (0.508 mm) nozzles arrayed in four holes at low throughput, about 0.15 ghm. The facing layer was a spunlaced 30 gsm 100% PP staple fiber. The neat 100 samples were single layer fabrics of PCP-01 that were meltblown using a 15" (38.1 cm) die, 0.020" (0.508 mm) nozzles, 2 rows, and 0.145 ghm.

**Table 1: PCP-01 meltblown onto facing layer of spunlaced PP/PET staple fibers.**

| Ex. | PCP-01 basis wt% | FL PP/PET basis wt% | Total Basis wt | PCP-01 BW: Total BW | PCP-01 BW: FL BW | Peak Load | PS | Retractive Force @ 50% | Peak Load @ 100%/ / Retractive Force @ 50% |
|---|---|---|---|---|---|---|---|---|---|
| 1 | 25 | 60 | 85 | 0.294 | 0.417 | 1.129 | 0.23 | 0.046 | 24.5 |
| (1.7) | | | | | | | | | |
| 2 | 50 | 60 | 110 | 0.455 | 0.833 | 1.241 | 0.175 | 0.113 | 11.0 |
| 1.1 | | | | | | | | | |
| 3 | 75 | 60 | 135 | 0.556 | 1.250 | 1.326 | 0.17 | 0.128 | 10.4 |
| 1.2 | | | | | | | | | |
| 4 | 100 | 60 | 160 | 0.625 | 1.667 | 2.183 | 0.13 | 0.293 | 7.5 |
| 1.3 | | | | | | | | | |

**Table 2: PCP-01 meltblown onto facing layer of spunlaced PP staple fibers.**

| Ex. | PCP-01 basis wt% | FL PP/PET basis wt% | Total Basis wt | PP-01 BW: Total BW | PCP-01 BW: FL BW | Peak Load | PS | Retractive Force @ 50% | Peak Load @ 100% / Retractive Force @ 50% |
|---|---|---|---|---|---|---|---|---|---|
| 5 | 25 | 60 | 85 | 0.294 | 0.417 | 2.472 | 0.317 | 0.0273 | 90.5 |
| (1.8) | | | | | | | | | |
| 6 | 50 | 60 | 110 | 0.455 | 0.833 | 2.189 | 0.177 | 0.097 | 22.6 |
| 1.4 | | | | | | | | | |
| 7 | 75 | 60 | 135 | 0.556 | 1.250 | 2.494 | 0.157 | 0.16 | 15.6 |
| 1.5 | | | | | | | | | |
| 8 | 100 | 60 | 160 | 0.625 | 1.667 | 3.355 | 0.13 | 0.333 | 10.1 |
| 1.6 | | | | | | | | | |

**Table 3: Neat PCP-01 meltblown layer (no facing layer).**

| Ex. | PCP-01 basis wt% | FL PP/PET basis wt% | Total Basis wt | PCP-01 BW: Total BW | PCP-01 BW: FL BW | Peak Load | PS | Retractive Force @ 50% | Peak Load @ 100% / Retractive Force @ 50% |
|---|---|---|---|---|---|---|---|---|---|
| Neat 100 | 100 | 0 | 100 | 1.000 | → ∞ | 0.81 | 0.11 | 0.267 | 3.0 |
| Neat 100 | 100 | 0 | 100 | 1.000 | → ∞ | 0.466 | 0.075 | 0.154 | 3.0 |

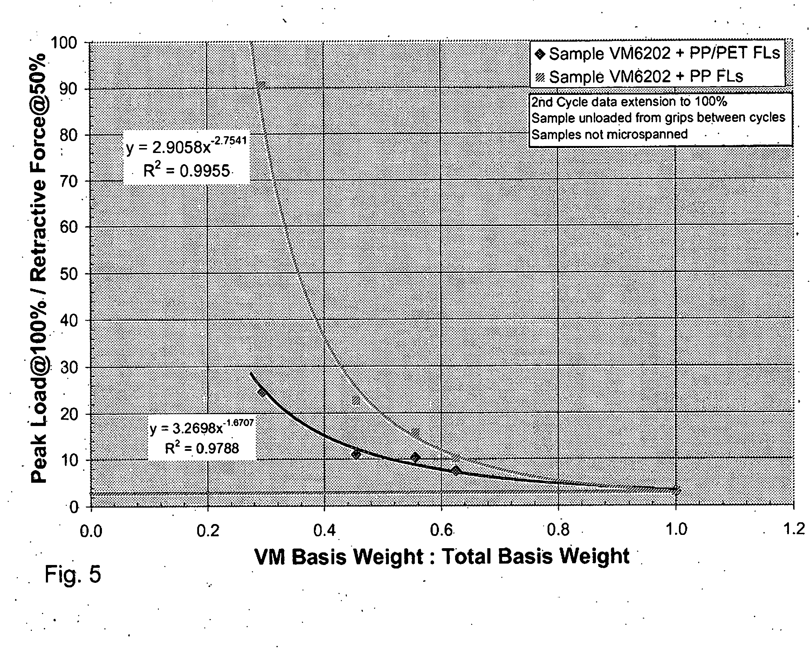

Figure 5 graphically depicts the samples reported in Tables 1-3 with the PCP-01 Basis Weight per Total Fabric Basis Weight on the x-axis and the peak force @ 100% / Retractive Force @ 50% on the y-axis. The data at 1.0 on the x-axis represents the neat 100 gsm PCP-01 fabric described in Table 3. This represents the asymptotic value that the tri-laminate should approach as the relative amounts of facing layers decrease and their constraint on the elastic meltblown PCP-01 approaches zero.

Figure 5 shows a very good fit to a power law relationship: y=Ax^{b} where A=3 and b was -1.67 for the soft stretch PP/PET examples (1-4) and b was -2.75 for the two facing layers for the PP examples (5-8). Surprisingly, the expression, y=Ax^{b}, where A=3, b = 0 → ∞, x = peak load @ 100% / Retractive Force @ 50% and y = facing layer constraint, is found to describe the general elastic performance of laminates containing the PCP-01 made using the multilayered construction process. It was surprisingly found that the ratio of the peak load at 100% strain on the second hysteresis cycle to the retractive force at 50% strain was a constant with a value of 3 for PP-01.

Still referring to Figure 5, it has been discovered that different facing layers introduce different levels of constraint as defined by the value of y. As the ratio of the PCP content to the overall fabric basis weight decreased as defined by the x value (i.e., less PCP for a constant facing layer construct) the ratio of peak force to retractive force increased. As the PCP content decreased for a constant facing layer construct, the level of constraint followed the general power law relationship described. Surprisingly, changing the nature of the facing layer changed the level of constraint; however, the general power law held, just the power changed.

The less constraint that the facing layer imparts, the lower the "b" value. Conversely, the greater the constraint imposed by the facing layer, the greater the "b" value. The two extreme cases being, if the facing layer imposed no constraint whatsoever on the laminate then b = 0 and the 'y' value would equal 3 just like the value of the neat PCP with no facing layer. The other extreme would be if the facing layers were highly constraining stiff plastic layers that once stretched to 100% did not allow any recovery of more than 50%. Therefore, if the retractive force at 50% is 0, the ratio is infinite and b approaches infinity.

Certain embodiments and features have been described using a set of numerical upper limits and a set of numerical lower limits. It should be appreciated that ranges from any lower limit to any upper limit are contemplated unless otherwise indicated. Certain lower limits, upper limits and ranges appear in one or more claims below. All numerical values are "about" or "approximately" the indicated value, and take into account experimental error and variations that would be expected by a person having ordinary skill in the art.

## Claims

1. A meltblown composite, comprising:
a first meltblown layer comprising one or more resins having an Ultimate Elongation (UE) of from about 50% to about 250%, as measured according to ASTM D412; and
a second meltblown layer comprising a propylene-α-olefin copolymer having an ethylene content of 5 wt% to 20 wt%; a MFR (ASTM-1238D, 2.16 kg, 230°C) of 10 g/10 min to 30 g/10 min; and a heat of fusion of 75 J/g or less.

2. The meltblown composite of claim 1, wherein the second meltblown layer comprises at least 60 wt% of the propylene-α-olefin copolymer, preferably wherein the second meltblown layer consists of the propylene-α-olefin copolymer.

3. The meltblown composite according to any claims 1 or 2, wherein the first meltblown, layer comprises homopolypropylene, polypropylene, polyethylene, or blends thereof, preferably wherein the first meltblown layer comprises at least 5 wt% of polypropylene.

4. The meltblown composite according to any claims 1 to 3, wherein the propylene-α-olefin copolymer has a MFR (ASTM D1238, 2.16 kg, 230°C) of 15 g/10 min to 20 g/10 min.

5. The meltblown composite according to any claims 1 to 4, wherein the ethylene content of the propylene-α-olefin copolymer ranges from 13 wt% to 16 wt%.

6. The meltblown composite according to any claims 1 to 5, wherein each meltblown layer has a basis weight within the range of from 10 g/m² to 150 g/m².

7. The meltblown composite according to any claims 1 to 5, wherein the first meltblown layer has a basis weight within the range of from 5 g/m² to 300 g/m² and the second meltblown layer has a basis weight within the range of from 15 g/m² to 150 g/m².

8. A method for forming a multilayer meltblown composite, comprising:
meltblowing a first material to form a first meltblown layer; and
meltblowing a second material on at least a portion of the first melt blown layer, wherein the second meltblown layer comprises a propylene-α-olefin copolymer having an ethylene content of 5 wt% to 20 wt%; a MFR (ASTM-1238D, 2.16 kg, 230°C) of 10 g/10 min to 30 g/10 min; and a heat of fusion of 75 J/g or less.

9. The method of claim 8, wherein each material is meltblown onto a moving surface.

10. The method of claims 8 or 9, wherein the second meltblown layer comprises at least 60 wt% of the propylene-α-olefm copolymer, preferably wherein the second meltblown layer consists essentially of the propylene-α-olefin copolymer.

11. The method according to any claims 8 to 10, wherein the first meltblown layer comprises homopolypropylene, polypropylene, polyethylene, or blends thereof, preferably wherein the first meltblown layer comprises at least 5 wt% of polypropylene.

12. The method according to any claims 8 to 11, wherein the propylene-α-olefin copolymer has a MFR (ASTM D1238, 2.16 kg, 230°C) of 15 g/10 min to 20 g/10 min.

13. The method according to any claims 8 to 12, wherein the ethylene content of the propylene-α-olefin copolymer ranges from 13 wt% to 16 wt%.

14. The method according to any claims 8 to 13, wherein each meltblown layer has a basis weight within the range of from 10 g/m² to 150 g/m².

15. The method according to any claims 8 to 13, wherein the first meltblown layer has a basis weight within the range of from 5 g/m² to 300 g/m² and the second meltblown layer has a basis weight within the range of from 15 g/m² to 150 g/m².

16. An article of manufacture, incorporating as one or more components, a multilayer meltblown composite according to claim 1.

## Patentansprüche

1. Schmelzgeblasene Verbundstruktur, die
eine erste schmelzgeblasene Schicht, die ein oder mehrere Harze mit einer Reißdehnung (Ultimate Elongation (UE)) von etwa 50% bis etwa 250%, gemessen gemäß ASTM D412, umfasst, und
eine zweite schmelzgeblasene Schicht, die eine Propylen/α-Olefin-Copolymer umfasst, das einen Ethylengehalt von 5 Gew.-% bis 20 Gew.%, einen MFR (ASTM-1238D, 2,16 kg, 230°C) von 10 g/10 Min. bis 30 g/10 Min. und eine Schmelzwärme von 75 J/g oder weniger aufweist,
umfasst.

2. Schmelzgeblasene Verbundstruktur gemäß Anspruch 1, bei der die zweite schmelzgeblasene Schicht mindestens 60 Gew.-% Propylen/a-Olefin-Copolymer umfasst, wobei die zweite schmelzgeblasene Schicht vorzugsweise aus dem Propylen/α-Olefin-Copolymer besteht.

3. Schmelzgeblasene Verbundstruktur gemäß einem der Ansprüche 1 oder 2, bei der die erste schmelzgeblasene Schicht Homopolypropylen, Polypropylen, Polyethylen oder Gemische davon umfasst, wobei die erste schmelzgeblasene Schicht vorzugsweise mindestens 5 Gew.-% Polypropylen umfasst.

4. Schmelzgeblasene Verbundstruktur gemäß einem der Ansprüche 1 bis 3, bei der das Propylen/a-Olefin-Copolymer einen MFR (ASTM D1238, 2,16 kg, 230°C) von 15 g/10 Min. bis 20 g/10 Min. aufweist.

5. Schmelzgeblasene Verbundstruktur gemäß einem der Ansprüche 1 bis 4, bei der der Ethylengehalt des Propylen/a-Olefin-Copolymers im Bereich von 13 Gew.-% bis 16 Gew.-% liegt.

6. Schmelzgeblasene Verbundstruktur gemäß einem der Ansprüche 1 bis 5, bei der jede schmelzgeblasene Schicht ein Basisgewicht innerhalb des Bereichs von 10 g/m² bis 150 g/m² aufweist.

7. Schmelzgeblasene Verbundstruktur gemäß einem der Ansprüche 1 bis 5, bei der die erste schmelzgeblasene Schicht ein Basisgewicht innerhalb des Bereichs von 5 g/m² bis 300 g/m² und die zweite schmelzgeblasene Schicht ein Basisgewicht innerhalb des Bereichs von 15 g/m² bis 150 g/m² aufweist.

8. **Verfahren zur Bildung einer mehrschichtigen** schmelzgeblasenen Verbundstruktur, bei dem
**ein erstes Material unter Bildung einer ersten** schmelzgeblasenen Schicht schmelzgeblasen wird und
ein zweites Material auf mindestens einen Teil der ersten schmelzgeblasenen Schicht schmelzgeblasen wird, wobei die zweite schmelzgeblasene Schicht ein Propylen/a-Olefin-Copolymer umfasst, das einen Ethylengehalt von 5 Gew.-% bis 20 Gew.-%, einen MFR (ASTM-1238D, 2,16 kg, 230°C) von 10 g/10 Min. bis 30 g/10 Min. und eine Schmelzwärme von 75 J/g oder weniger aufweist.

9. Verfahren nach Anspruch 8, bei dem jedes Material auf eine bewegende Oberfläche schmelzgeblasen wird.

10. Verfahren nach Anspruch 8 oder 9, bei dem die zweite schmelzgeblasene Schicht mindestens 60 Gew.-% des Propylen/α-Olefin-Copolymers umfasst, wobei die zweite schmelzgeblasene Schicht vorzugsweise aus dem Propylen/α-Olefin-Copolymer besteht.

11. Verfahren nach einem der Ansprüche 8 bis 10 bei dem die erste schmelzgeblasene Schicht Homopolypropylen, Polypropylen, Polyethylen oder Gemische davon umfasst, wobei die erste schmelzgeblasene Schicht vorzugsweise mindestens
5 Gew.-% Polypropylen umfasst.

12. Verfahren gemäß einem der Ansprüche 8 bis 11, bei dem das Propoylen/α-Olefin-Copolymer einen MFR (ASTM D1238, 2,16 kg, 230°C) von 15 g/10 Min. bis 20 g/10 Min. aufweist.

13. Verfahren gemäß einem der Ansprüche 8 bis 12 bei dem der Ethylengehalt des Propylen/a-Olefin-Copolymers im Bereich von 13 Gew.-% bis 16 Gew.-% liegt.

14. Verfahren gemäß einem der Ansprüche 8 bis 13, bei dem jede schmelzgeblasene Schicht ein Basisgewicht innerhalb des Bereichs von 10 g/m² bis 150 g/m² aufweist.

15. Verfahren gemäß einem der Ansprüche 8 bis 13, bei dem die erste schmelzgeblasene Schicht vorzugsweise ein Basisgewicht innerhalb des Bereichs von 5 g/m² bis 300 g/m² und die zweite schmelzgeblasene Schicht vorzugsweise ein Basisgewicht innerhalb des Bereichs von 15 g/m² bis 150 g/m² aufweist.

16. Fertigungsgegenstand, der als eine oder mehrere Komponenten eine mehrschichtige schmelzgeblasene Verbundstruktur gemäß Anspruch 1 umfasst.

## Revendications

1. Composite extrudé-soufflé, comprenant :
une première couche extrudée-soufflée comprenant une ou plusieurs résines ayant un allongement à la rupture de 50 % à 250 %, mesuré selon ASTM D412 ; et
une deuxième couche extrudée-soufflée comprenant un copolymère de propylène-α-oléfine ayant une teneur en éthylène de 5 % en poids à 20 % en poids ; un indice d'écoulement à l'état fondu (ASTM-1238D, 2,16 kg, 230 °C) de 10 g/10 min à 30 g/10 min ; et une chaleur de fusion de 75 J/g ou moins.

2. Composite extrudé-soufflé selon la revendication 1, dans lequel la deuxième couche extrudée-soufflée comprend au moins 60 % en poids du copolymère de propylène-α-oléfine, préférablement dans lequel la deuxième couche extrudée-soufflée se compose du copolymère de propylène-α-oléfine.

3. Composite extrudé-soufflé selon l'une quelconque des revendications 1 ou 2, dans lequel la première couche extrudée-soufflée comprend un homopolypropylène, un polypropylène, un polyéthylène, ou des mélanges de ceux-ci, préférablement dans lequel la première couche extrudée-soufflée comprend au moins 5 % en poids de polypropylène.

4. Composite extrudé-soufflé selon l'une quelconque des revendications 1 à 3, dans lequel le copolymère de propylène-α-oléfine a un indice d'écoulement à l'état fondu (ASTM-D1238, 2,16 kg, 230 °C) de 15 g/10 min à 20 g/10 min.

5. Composite extrudé-soufflé selon l'une quelconque des revendications 1 à 4, dans lequel la teneur en éthylène du copolymère de propylène-α-oléfine est de 13 % en poids à 16 % en poids.

6. Composite extrudé-soufflé selon l'une quelconque des revendications 1 à 5, dans lequel chaque couche extrudée-soufflée a un grammage de 10 g/m² à 150 g_{/}m².

7. Composé extrudé-soufflé selon l'une quelconque des revendications 1 à 5, dans lequel la première couche extrudée-soufflée a un grammage de 5 g/m² à 300 g/m² et la deuxième couche extrudée-soufflée a un grammage de 15 g/m² à 150 g/m².

8. Procédé de formation d'un composite extrudé-soufflé multicouche, consistant à :
extruder-souffler un premier matériau pour former une première couche extrudée-soufflée ; et
extruder-souffler un deuxième matériau sur au moins une partie de la première couche extrudée-soufflée, la deuxième couche extrudée-soufflée comprenant un copolymère de propylène-α-oléfine ayant une teneur en éthylène de 5 % en poids à 20 % en poids ; un indice d'écoulement à l'état fondu (ASTM-1238D, 2,16 kg, 230 °C) de 10 g/10 min à 30 g/10 min ; et une chaleur de fusion de 75 J/g ou moins.

9. Procédé selon la revendication 8, dans lequel chaque matériau est extrudé-soufflé sur une surface en mouvement.

10. Procédé selon les revendications 8 ou 9, dans lequel la deuxième couche extrudée-soufflée comprend au moins 60 % en poids du copolymère de propylène-α-oléfine, préférablement dans lequel la deuxième couche extrudée-soufflée se compose du copolymère de propylène-α-oléfine.

11. Procédé selon l'une quelconque des revendications 8 à 10, dans lequel la première couche extrudée-soufflée comprend un homopolypropylène, un polypropylène, un polyéthylène, ou des mélanges de ceux-ci, préférablement dans lequel la première couche extrudée-soufflée comprend au moins 5 % en poids de polypropylène.

12. Procédé selon l'une quelconque des revendications 8 à 11, dans lequel le copolymère de propylène-α-oléfine a un indice d'écoulement à l'état fondu (ASTM-D1238, 2,16 kg, 230 °C) de 15 g/10 min à 20 g/10 min.

13. Procédé selon l'une quelconque des revendications 8 à 12, dans lequel la teneur en éthylène du copolymère de propylène-α-oléfine est de 13 % en poids à 16 % en poids.

14. Procédé selon l'une quelconque des revendications 7 à 12, dans lequel chaque couche extrudée-soufflée a un grammage de 10 g/m² à 150 g/m².

15. Procédé selon l'une quelconque des revendications 7 à 12, dans lequel la première couche extrudée-soufflée a un grammage de 5 g/m² à 300 g/m² et la deuxième couche extrudée-soufflée a un grammage de 15 g/m² à 150 g/m².

16. Produit manufacturé intégrant, sous la forme d'un ou plusieurs constituants, un composite extrudé-soufflé multicouche selon la revendication 1.
